# EUROPEAN PATENT APPLICATION

(11) **EP 4 831 273 A1**
(43) Date of publication of application: **28.10.2026**
(21) Application number: 24907498.0
(22) Date of filing: 19.12.2024
(51) Int. Cl.: C12M 3/00, C12N 5/074, C12N 5/0735

(54) **STEM CELL ADHESION SHEET**

(30) Priority: 21.12.2023 JP 2023216225
(71) Applicant: Fujita Academy, Toyoake-shi, Aichi 470-1192 (JP)
(72) Inventor: HORI, Hideo, Toyoake-shi, Aichi 470-1192 (JP); FUJITA, Nobuyuki, Toyoake-shi, Aichi 470-1192 (JP); YABUUCHI, Hikaru, Toyoake-shi, Aichi 470-1192 (JP); OBATA, Tsutomu, Takarazuka-shi, Hyogo 665-0824 (JP)
(74) Representative: Heyerhoff Geiger GmbH & Co. KG
(86) International application number: PCT/JP2024/044974
(87) International publication number: WO 2025/135114

(57) **Abstract**

An object is to provide a stem cell adhesion sheet that can stably retain stem cells. A stem cell adhesion sheet includes a nonwoven fabric, the nonwoven fabric has a laminated structure including a first layer formed of fibers mainly composed of a biocompatible polymer and a second layer formed of fibers mainly composed of a biocompatible polymer and formed of fibers having a larger fiber diameter than a fiber forming the first layer, and the biocompatible polymer forming the first layer and the biocompatible polymer forming the second layer contain the same type of polymer material.

## Description

### [Technical Field]

The present disclosure of the present application relates to a stem cell adhesion sheet.

### [Background Art]

In recent years, in the field of regenerative medicine, development of tissue regeneration promoting materials that can retain cells therein and facilitates tissue regeneration by being implanted in bodies has been progressed. For example, Patent Literature 1 discloses a sheet-like tissue regeneration promoting material having a surface on which at least one of a group consisting of white blood cells and platelets is present. Further, Patent Literature 2 discloses a liquid tissue regeneration promoting material obtained by mixing powder of a polymer material with cells.

### [Citation List]

### [Patent Literature]

Patent Literature 1: Japanese Patent No. 4847129
Patent Literature 2: Japanese Patent Application Laid-Open No. 2015-112262

### [Summary of Invention]

### [Technical Problem]

In the tissue regeneration promoting materials disclosed in Patent Literatures 1 and 2, however, since powder of a porous material or a polymer material is used, there is a problem that it is difficult to stably retain mesenchymal stem cells having a high tissue regenerative ability among cells. Such a problem exists not only in a case where mesenchymal stem cells are retained by a tissue regeneration promoting material but also in a case where other stem cells are retained.

The disclosure of the present application has been made based on such a background and intends to provide a stem cell adhesion sheet that can stably retain stem cells.

### [Solution to Problem]

The disclosure of the present application relates to a stem cell adhesion sheet illustrated below.
(1) A stem cell adhesion sheet comprising a nonwoven fabric,
   wherein the nonwoven fabric has a laminated structure including
   a first layer formed of fibers mainly composed of a biocompatible polymer, and
   a second layer formed of fibers mainly composed of a biocompatible polymer and formed of fibers having a larger fiber diameter than fibers forming the first layer, and
   wherein the biocompatible polymer forming the first layer and the biocompatible polymer forming the second layer contain the same type of polymer material.
(2) The stem cell adhesion sheet according to (1) above, wherein the polymer material is of at least one type selected from a group consisting of polylactic acid, polycaprolactone, chitin, chitosan, polyglycolic acid, and a copolymer thereof.
(3) The stem cell adhesion sheet according to (2) above, wherein the polymer material is at least one type selected from a group consisting of polylactic acid, polycaprolactone, and a copolymer of polylactic acid and polycaprolactone.
(4) The stem cell adhesion sheet according to (1) above, wherein polymer materials of the biocompatible polymer forming the first layer and the biocompatible polymer forming the second layer contain the same type of polymer material at a ratio of at least 25% or higher.
(5) The stem cell adhesion sheet according to (2) above, wherein polymer materials of the biocompatible polymer forming the first layer and the biocompatible polymer forming the second layer contain the same type of polymer material at a ratio of at least 25% or higher.
(6) The stem cell adhesion sheet according to (3) above, wherein polymer materials of the biocompatible polymer forming the first layer and the biocompatible polymer forming the second layer contain the same type of polymer material at a ratio of at least 25% or higher.
(7) The stem cell adhesion sheet according to any one of (4) to (6) above, wherein the polymer materials of the biocompatible polymer forming the first layer and the biocompatible polymer forming the second layer are composed of the same type of material at 100%.
(8) The stem cell adhesion sheet according to any one of (1) to (7) above,
   wherein the fiber diameter of the fibers forming the first layer is in a range of 200 nm or greater and 5 µm or less, and
   wherein the fiber diameter of the fibers forming the second layer is larger than the fiber diameter of the fibers forming the first layer and is in a range of 30 µm or less.
(9) The stem cell adhesion sheet according to any one of (1) to (8) above,
   wherein a basis weight of the first layer is in a range of 0.2 g/m² or greater and 4 g/m² or less, and
   wherein a basis weight of the second layer is in a range of 2 g/m² or greater and 30 g/m² or less.
(10) The stem cell adhesion sheet according to (3) above,
   wherein inclusion of siloxane in the biocompatible polymer is excluded, and
   wherein the fiber diameter of the fibers forming the second layer is 3.2 µm to 15 µm excluding that the fiber diameter is 4.0 µm or less and that the fiber diameter is 10 µm or greater.
(11) The stem cell adhesion sheet according to (1) above,
   wherein inclusion of siloxane in the biocompatible polymer is excluded,
   wherein the fiber diameter of the fibers forming the first layer is 200 nm or greater and 5 µm or less excluding that the fiber diameter is 1.5 µm or greater, and
   wherein the fiber diameter of the fibers forming the second layer is 3.2 µm to 15 µm excluding that the fiber diameter is 4.0 µm or less and that the fiber diameter is 10 µm or greater.
(12) The stem cell adhesion sheet according to (1) above,
   wherein inclusion of siloxane in the biocompatible polymer is excluded,
   wherein a basis weight of the first layer is in a range of 0.2 g/m² or greater and 4 g/m² or less, and
   wherein the fiber diameter of the fibers forming the second layer is 3.2 µm to 15 µm excluding that the fiber diameter is 4.0 µm or less and that the fiber diameter is 10 µm or greater.

### [Advantageous Effects of Invention]

The stem cell adhesion sheet disclosed in the present application makes it possible to stably retain stem cells.

### [Brief Description of Drawings]

[FIG. 1]

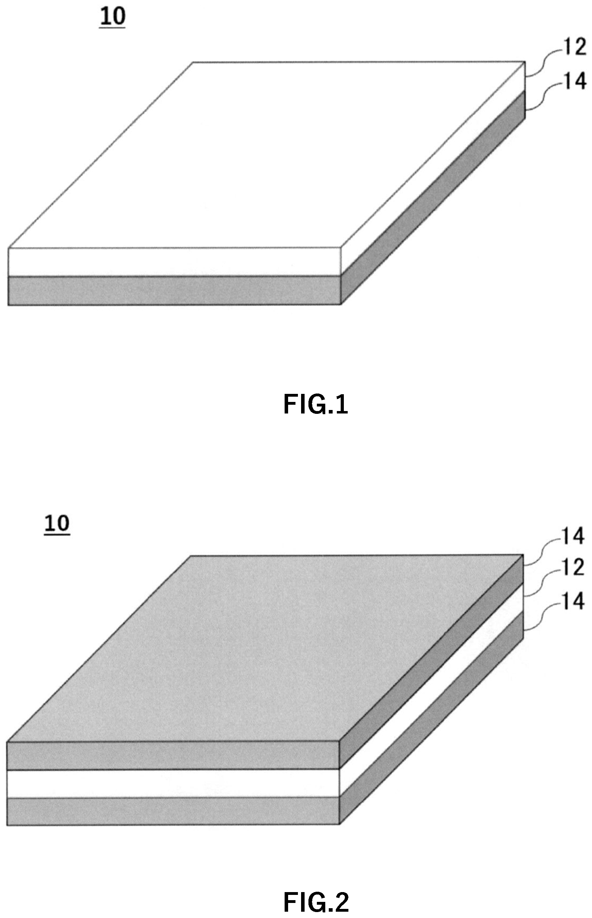

   FIG. 1 is a perspective view illustrating an example configuration of a stem cell adhesion sheet according to an embodiment.
[FIG. 2]
   FIG. 2 is a perspective view illustrating an example configuration of a stem cell adhesion sheet according to an embodiment.
[FIG. 3]

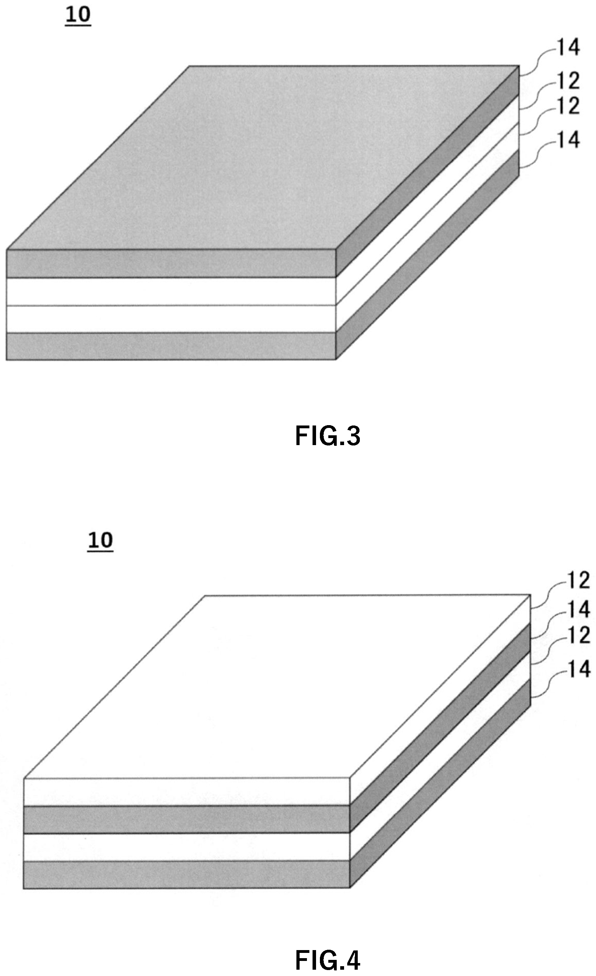

   FIG. 3 is a perspective view illustrating an example configuration of a stem cell adhesion sheet according to an embodiment.
[FIG. 4]
   FIG. 4 is a perspective view illustrating an example configuration of a stem cell adhesion sheet according to an embodiment.
[FIG. 5]

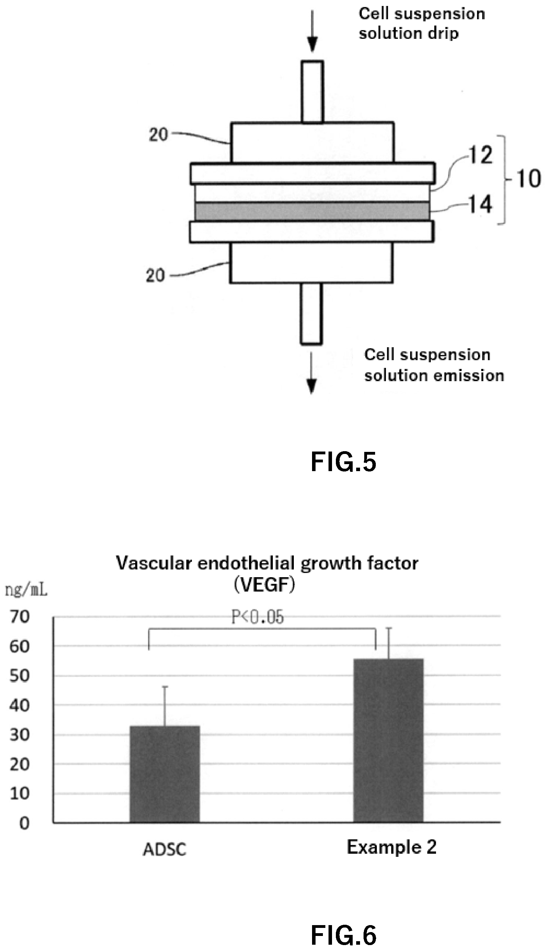

   FIG. 5 is a front view illustrating an overview of preparing a tissue regeneration promoting sheet by using the stem cell adhesion sheet according to the embodiment.
[FIG. 6]
   FIG. 6 is a graph illustrating a difference in the expression level of vascular endothelial growth factor (VEGF) produced by human adipose-derived mesenchymal stem cells when a tissue regeneration promoting sheet prepared in Example 2 was used and when no tissue regeneration promoting sheet is used.
[FIG. 7]

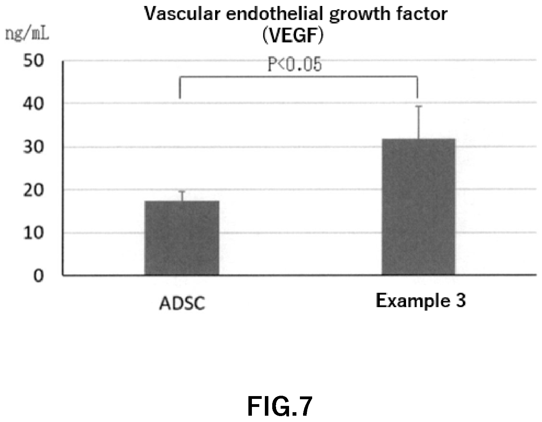

   FIG. 7 is a graph illustrating a difference in the expression level of vascular endothelial growth factor (VEGF) produced by human adipose-derived mesenchymal stem cells when a tissue regeneration promoting sheet prepared in Example 3 was used and when no tissue regeneration promoting sheet is used.

### [Description of Embodiments]

A stem cell adhesion sheet disclosed in the present application will be described below with reference to the drawings. Note that the position, size, range, or the like of each configuration illustrated in the drawings do not always represent the actual position, size, range, or the like in order to facilitate the understanding. Thus, the disclosure of the present application is not necessarily limited to the position, size, range, or the like disclosed in the drawings.

Further, in the present specification, it is construed that:
(1) numerical ranges expressed by using "to" mean a range including numerical values stated before and after "to" as the lower limit value and the upper limit value;
(2) numerical values, numerical ranges, and qualitative expressions (for example, expressions such as "identical", "same", or the like) represent the numerical values, numerical ranges, and properties including errors generally accepted in this technical field; and
(3) when the term "substantially XX shape" is stated, a shape recognized as not only accurately XX shape but also approximately XX shape is included.

### [Embodiment of stem cell adhesion sheet]

The overview of a stem cell adhesion sheet 10 according to an embodiment will be described with reference to FIG. 1 to FIG. 4. FIG. 1 to FIG. 4 are perspective views illustrating example configurations of the stem cell adhesion sheet according to the embodiment.

The stem cell adhesion sheet according to the embodiment is used to attach stem cells thereto. A sheet resulted after stem cells have been attached thereto (hereafter, which may be referred to as "tissue regeneration promoting sheet") has an advantageous effect of promoting tissue regeneration at a damaged site by being implanted in the damaged site in a body. The stem cell adhesion sheet according to the embodiment includes a nonwoven fabric formed of fibers having a small diameter that allows attachment of stem cells thereto, for example, microfibers. A nonwoven fabric is a sheet in which fibers having a small diameter are entangled to each other without being woven. A nonwoven fabric can be freely deformed into any shape and thus is preferable to be embedded in or adhered to a damaged site in accordance with the shape of the damaged site. The tissue regeneration promoting sheet can be adhered to or embedded in any organs, for example, heart, blood vessels, lungs, trachea, esophagus, stomach, small intestine, large intestine, liver, kidneys, bladder, bones, muscles, and skin. The tissue regeneration promoting sheet can be caused to function as a scaffold that promotes proliferation of particular cells in a body.

As illustrated in FIG. 1, the stem cell adhesion sheet 10 includes a first layer 12 formed of fibers mainly composed of a biocompatible polymer and a second layer 14 formed of fibers mainly composed of a biocompatible polymer. The second layer 14 is formed of fibers having a larger fiber diameter than fibers forming the first layer 12.

Stem cells are captured in the stem cell adhesion sheet 10 by being attached to fibers rather than being trapped in a spacing between fibers forming a nonwoven fabric, as illustrated in FIG. 3 of Japanese Patent Application Laid-Open No. 2023-47494. While a procedure of attachment of stem cells to the stem cell adhesion sheet 10 will be described later, stem cells can be attached to both the first layer 12 and the second layer 14. Further, the second layer 14 is formed of thicker fibers than the fiber diameter of the fibers forming the first layer 12 and, thus, not only attaches stem cells but also functions as a reinforcement sheet to reinforce the first layer 12. If only the first layer 12 having a smaller fiber diameter is used to form a stem cell adhesion sheet, such a stem cell adhesion sheet will be likely to curl up when exposed to the cell suspension solution. In the stem cell adhesion sheet 10 according to the embodiment, lamination of the first layer 12 and the second layer 14 improves ease of handling of the stem cell adhesion sheet 10. The first layer 12 and the second layer 14 only need to be laminated so as not to easily separate from each other. The first layer 12 and the second layer 14 may be joined to each other by mutual entanglement of fibers during manufacturing of a nonwoven fabric, or the first layer 12 and the second layer 14 that have been separately prepared may be joined to each other by an adhesive agent or thermal fusion, though not limited thereto.

Although FIG. 1 illustrates an example of the stem cell adhesion sheet 10 in which one first layer 12 and one second layer 14 are laminated, the number of first layers 12 and the number of second layers 14 are not particularly limited as long as they are in a range that enables filtration of cell suspension solution containing stem cells and attachment of the stem cells to the stem cell adhesion sheet 10.

For example, as illustrated in FIG. 2, a single first layer 12 may be laminated between two top and bottom second layers 14. Further, as illustrated in FIG. 3, a plurality of (in the example illustrated in FIG. 3, two) first layers 12 may be laminated between two top and bottom second layers 14. Further, as illustrated in FIG. 4, first layers 12 and second layers 14 may be alternatingly laminated.

The first layer 12 and the second layer 14 are formed of fibers mainly composed of biocompatible polymers. Note that, in the present specification, "biocompatible polymer" means polymers having affinity with biological tissues or organs and having properties not causing a foreign body reaction, a rejection, or the like when applied to a living body.

The polymer material used in preparing the biocompatible polymer is not particularly limited as long as it exhibits high biocompatibility. The polymer material may be, but not limited to, polylactic acid (PLA), polycaprolactone (PCL), chitin, chitosan, polyglycolic acid (PGA), copolymers thereof, or the like. However, for the polymer material used for preparing the biocompatible polymer, siloxane may be absent. In other words, the biocompatible polymer may be polymers not including siloxane.

Among the polymer materials listed above as examples, polycaprolactone is semi-crystalline biodegradable thermoplastic polyester and can also be suitably used for manufacturing of nonwoven fabrics. Polycaprolactone may be, for example, poly(ε-caprolactone). The molecular weight of polycaprolactone may be a molecular weight suitable for injection molding and may be in a range of 70,000 to 200,000, for example. Further, the molecular weight of polylactic acid may be 50,000 to 200,000, and the molecular weight of polyglycolic acid may be 50,000 to 500,000.

The fibers forming the first layer 12 and the second layer 14 may be composed of only the biocompatible polymer formed of the above polymer materials or may contain other substances. As other substances, for example, agents promoting tissue regeneration or other biodegradable polymers may be used. The agents promoting tissue regeneration may be, for example, hydroxyapatite (calcium hydroxyphosphate), tricalcium phosphate, or the like. Other biodegradable polymers may be, for example, polydioxanone or the like. Note that the ratio of the mass of the biocompatible polymer in the fibers may be 50% or higher, 60% or higher, 70% or higher, 80% or higher, 90% or higher, 100%, or the like.

Although not yet approved in Japan, nonwoven fabrics prepared with 100% polylactic acid (PLA) have been approved for medical use in the United States. Further, in Japan, a copolymer consisting of 50% polycaprolactone (PCL) and 50% polylactic acid (PLA) has been approved for medical use as a synthetic artificial dura mater (trade name: Seamdura, manufactured by Gunze), and a copolymer consisting of 25% polycaprolactone (PCL) and 75% polylactic acid (PLA) has been approved for medical use as an absorbable suture (trade name: P(LA/CL) suture, manufactured by Gunze). That is, it can be said that polylactic acid and polycaprolactone are polymer materials whose safety in humans has been confirmed. Therefore, as the polymer material, polylactic acid, polycaprolactone, and copolymers of polylactic acid and polycaprolactone are more preferable in terms of reduction in the burden of filing a pharmaceutical approval application including safety testing when the polymer material is applied to a living body.

It is preferable for the biocompatible polymer forming the first layer 12 and the second layer 14 to contain the same type of polymer material. Once the tissue regeneration promoting sheet is implanted in a damaged site in a living body, the biocompatible polymer is degraded in the living body. In this process, if the first layer 12 and the second layer 14 differ in the polymer material, this may cause a difference to occur in environment at the implantation site, such as a degradation rate, a degraded product, pH, or the like. When the biocompatible polymers forming the first layer 12 and the second layer 14 contain the same type of polymer material, a smaller difference is expected in the environmental change after degradation at the implantation site.

It is expected that a larger ratio of the same type of polymer material will increase an obtained effect. The ratio of the same type of polymer material in the polymer material of the biocompatible polymer forming the first layer 12 and the second layer 14 may be, but not limited to, 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, 95% or higher, 100%, or the like.

Note that, in the present specification, the ratio of the same type of polymer material means the ratio of the smaller mass out of the masses of a component common to the first layer 12 and the second layer 14 in comparison between the mass of a component where the mass of the polymer material of the first layer 12 is defined as 100 and the mass of a component where the mass of the polymer material of the second layer 14 is defined as 100. Further, when the polymer materials are copolymers, the ratio of the smaller mass of the component can be defined as the ratio of the same type of polymer material in comparison between the masses of the same type of components contained in the copolymers. The number of same types may be one or two or greater. When two or more types of polymer materials (components) of the same type are contained, the ratios thereof can be summed. More specific examples when the polymer materials are X, Y, and Z are illustrated below. Note that the denotation of "X + Y" means a copolymer of X and Y.

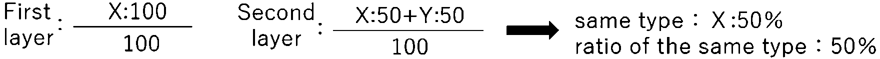

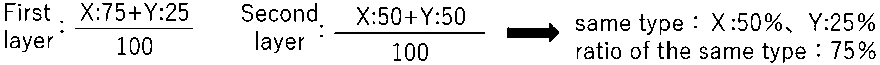

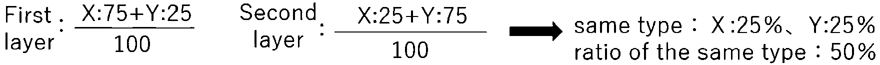

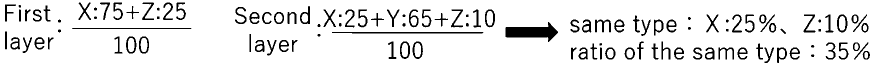

The fiber diameter of the fibers forming the first layer 12 and the fiber diameter of the fibers forming the second layer 14 are not particularly limited provided that the fiber diameter of the fibers forming the first layer 12 is smaller than the fiber diameter of the fibers forming the second layer 14, that stem cells can be attached to the fibers, and that there is no problem in handling of the stem cell adhesion sheet. The lower limit value of the fiber diameter of the fibers forming the first layer 12 may be, but not limited to, 200 nm or greater, 300 nm or greater, 400 nm or greater, 500 nm or greater, 600 nm or greater, 700 nm or greater, 800 nm or greater, 900 nm or greater, 1 µm or greater, or the like. On the other hand, the upper limit value thereof may be 5 µm or less, 4.75 µm or less, 4.5 µm or less, 4.25 µm or less, 4 µm or less, or the like. The lower limit value of the fiber diameter of the fibers forming the second layer 14 can be any value as long as it is greater than the fiber diameter of the fibers forming the first layer 12, and the upper limit value thereof may be 30 µm or less, 27.5 µm or less, 25 µm or less, 22.5 µm or less, 20 µm or less, 17.5 µm or less, 15 µm or less, or the like. Further, when the fiber diameter of the fibers forming the first layer 12 is defined as 1, the fiber diameter of the fibers forming the second layer 14 may be, but not limited to, 1.2 times or greater, 1.4 times or greater, 1.6 times or greater, 1.8 times or greater, 2 times or greater, 3 times or greater, 4 times or greater, 5 times or greater, 6 times or greater, 7 times or greater, 8 times or greater, 9 times or greater, 10 times or greater, or the like. Note that the fiber diameters listed above are mere examples and may be expressed by a range of a lower limit value and an upper limit value by using any numerical values that are not listed as the examples. The fiber diameter of the fibers forming the first layer 12 may be expressed such as, for example, greater than or equal to 200 nm and less than 1.5 µm as long as it is within a range greater than or equal to 200 nm and less than or equal to 5 µm. Further, the fiber diameter of the fibers forming the second layer 14 may be expressed such as, for example, greater than 4.0 µm and less than 10 µm as long as it is within a range greater than 200 nm and less than or equal to 30 µm.

For the first layer 12, a greater basis weight makes it possible to increase the quantity of stem cells to be attached but makes it gradually difficult to filter a cell suspension solution. The basis weight of the first layer 12 can be determined taking the quantity of stem cells to be attached and the ease of manufacturing into consideration. The lower limit value of the basis weight of the first layer 12 may be, but not limited to, 0.2 g/m² or greater, 0.4 g/m² or greater, 0.6 g/m² or greater, 0.8 g/m² or greater, 1 g/m² or greater, or the like, and the upper limit value thereof may be, but not limited to, 4 g/m² or less, 3 g/m² or less, 2.8 g/m² or less, 2.6 g/m² or less, 2.4 g/m² or less, 2.2 g/m² or less, 2.0 g/m² or less, or the like.

On the other hand, the second layer 14 not only attaches stem cells but also functions as a reinforcement sheet to reinforce the first layer 12, as described above. Therefore, the range of the basis weight of the second layer 14 can be determined as appropriate taking the ease of handling in addition to attachment of stem cells into consideration. The lower limit value thereof may be, but not limited to, 2 g/m² or greater, 2.5 g/m² or greater, 3 g/m² or greater, 3.5 g/m² or greater, 4 g/m² or greater, 4.5 g/m² or greater, 5 g/m² or greater, 5.5 g/m² or greater, 6 g/m² or greater, 6.5 g/m² or greater, 7 g/m² or greater, 7.5 g/m² or greater, 8 g/m² or greater, 8.5 g/m² or greater, 9 g/m² or greater, 9.5 g/m² or greater, 10 g/m² or greater, or the like, and the upper limit value thereof may be, but not limited to 30 g/m² or less, 29 g/m² or less, 28 g/m² or less, 27 g/m² or less, 26 g/m² or less, 25 g/m² or less, 24 g/m² or less, 23 g/m² or less, 22 g/m² or less, 21 g/m² or less, 20 g/m² or less, or the like. Note that, for the fiber diameters, the fiber diameter of the fibers forming the second layer 14 is required to be larger than the fiber diameter of the fibers forming the first layer 12. On the other hand, for the basis weights, the basis weight of the first layer 12 may be less than, the same as, or greater than the basis weight of the second layer 14 as long as it provides good handling and does not prevent filtration of stem cells.

The thickness of the laminated structure in which the first layer 12 and the second layer 14 are laminated can be determined as appropriate taking ease of handling with tweezers into consideration. The thickness of the laminated structure may be, for example, but not limited to 200 µm or greater, 300 µm or greater, 400 µm or greater, 500 µm or greater, 600 µm or greater, 700 µm or greater, or the like. On the other hand, the upper limit of the thickness of the laminated structure may be 1700 µm or less, 1600 µm or less, 1500 µm or less, 1400 µm or less, or the like. Further, since the second layer 14 has a function of a reinforcement sheet, there is no problem in the ease of handling even when the first layer 12 is thin. The thickness of the first layer 12 in the laminated structure described above may be 10 µm or greater, 20 µm or greater, 40 µm or greater, 60 µm or greater, 80 µm or greater, 100 µm or greater, or the like, and the upper limit value thereof may be less than 200 µm, 180 µm or less, 160 µm or less, 140 µm or less, or the like.

Stem cells to be attached to the stem cell adhesion sheet 10 may be mesenchymal stem cells (MSCs) or cells differentiated from mesenchymal stem cells, embryonic stem (ES) cells, induced pluripotent stem (iPS) cells, or the like. The mesenchymal stem cells are somatic stem cells having the ability to differentiate into cells belonging to the mesenchymal lineage and may be any of adipose-derived cells, periosteum-derived cells, synovium-derived cells, cancellous bone-derived cells, bone marrow-derived cells, amnion-derived cells, umbilical cord blood-derived cells, or placenta-derived cells. Stem cells to be attached to a nonwoven fabric may be of one type or may be of two or more types in combination.

The stem cells to be attached to the stem cell adhesion sheet 10 may be collected from a patient or may be established as a cell line. To culture stem cells in vitro, for example, a culture medium containing fetal bovine serum (FBS) or human serum, preferably human serum derived from a patient or serum-free culture medium can be used. Stem cells cultured in the culture medium may be attached directly to a nonwoven fabric or may be attached to a nonwoven fabric after stem cells are differentiated to express a particular type of cells, for example, osteoblasts. To express a particular type of cells, for example, a differentiation-inducing factor can be added to a culture medium of stem cells.

Further, it is not necessarily required to attach stem cells in advance to the stem cell adhesion sheet 10 disclosed in the present application. The stem cell adhesion sheet 10 may be arranged at a damaged site in a body, a cell suspension solution containing stem cells may then be administered to the vascular system, and thereby the stem cells may be attached to the stem cell adhesion sheet in the body.

Next, a specific procedure to attach stem cells to the stem cell adhesion sheet 10 according to the embodiment will be described. To attach stem cells to the stem cell adhesion sheet 10, a cell suspension solution containing stem cells can be in contact with the stem cell adhesion sheet 10 for a while, however, it is preferable to perform the following procedure in order to suppress a reduction in the growth factor production ability during culture of stem cell.

First, as illustrated in FIG. 5, the stem cell adhesion sheet 10 according to the embodiment is set between a pair of holders 20 forming a filtration device. Each holder 20 is configured such that the cell suspension solution can flow out of or flow into the holder 20. In the example illustrated in FIG. 5, when a cell suspension solution flows from one holder 20 to the other holder 20, the cell suspension solution comes into contact with the first layer 12.

Next, the cell suspension solution containing stem cells is dripped dropwise from above slowly one drop at a time, and the cell suspension solution is filtered at the first layer 12. The filtration rate may be, for example, a rate at which 1 ml of the cell suspension solution is dripped over 2 to 3 minutes. At this time, stem cells contained in the dripped cell suspension solution attach to fibers of the first layer 12 by themselves. Although the precise mechanism is unknown, once a cell suspension solution containing stem cells is filtered little by little through the stem cell adhesion sheet, the stem cells are stably attached to each fiber, the attached stem cells are activated, and thereby production of growth factors is promoted. Note that, while stem cells that have passed through the first layer 12 without attachment thereto may attach to the second layer 14, all the stem cells may be caused to attach to the first layer 12 via adjustment of conditions or the like. That is, while the second layer 14 has an adhesion function of stem cells, attachment of stem cells to the second layer 14 is not essential when the second layer 14 is used as the tissue regeneration promoting sheet.

The embodiments described above are examples, the present invention is not limited thereto, and various embodiments are possible within the scope not departing from the spirit of the invention recited in the claims. The components described in the embodiments can be freely combined with each other. Further, the invention equivalent to the invention recited in the claims is also included in the present invention.

For example, the stem cell adhesion sheet 10 illustrated in FIG. 5 may be any of the stem cell adhesion sheets 10 illustrated in FIG. 2 to FIG. 4, though not depicted. Further, the cell suspension solution may flow from the second layer 14 side. Further, although laminated structures having three or more layers are illustrated in the examples illustrated in FIG. 2 to FIG. 4, the number of types of the layers is two of the first layer 12 and the second layer 14. Alternatively, a third layer that differs in the composing polymer material, the fiber diameter size, or the like from the first layer 12 and the second layer 14 may be laminated. As long as the first layer 12 and the second layer 14 disclosed in the present application are included, other layers may be included.

Although Examples are presented below to specifically describe the embodiment disclosed in the present application, these Examples are provided merely to illustrate the embodiment. These Examples express neither limitation nor restriction of the technical scope disclosed in the present application.

### [EXAMPLES]

### <Example 1>

### [Preparation of various nonwoven fabrics]

A material obtained by copolymerizing polycaprolactone (PCL) and polylactic acid (PLA) at a ratio of 50:50, which has already been used clinically as artificial dura mater, was used as the polymer materials of the first layer 12 and the second layer 14. Nonwoven fabrics having fiber diameters, basis weights, and thicknesses listed in Table 1 below were prepared by using a commercially available nonwoven fabric preparation device.

**[Table 1]**

| First layer fiber diameter (*µ*m) | First layer basis weight (g/m²) | First layer thickness (*µ*m) | Second layer fiber diameter (*µ*m) | Second layer basis weight (g/m²) | Second layer thickness (*µ*m) | Number of laminated layers | Capture rate(%) |
|---|---|---|---|---|---|---|---|
| 0.34 | 1.94 | 97 | 4.31 | 10 | 500 | 1+ 1 | 100 ± 0.0 |
| 0.5 | 2.43 | 122 | 4.04 | 9.76 | 488 | 1+ 1 | 100 ± 0.0 |
| 0.81 | 2.22 | 111 | 3.96 | 9.76 | 488 | 1+ 1 | 100 ± 0.0 |
| 1.11 | 2.22 | 111 | 4.48 | 10 | 500 | 1+ 1 | 99.3 ± 1.0 |
| 3.09 | 2.22 | 111 | 4.23 | 10 | 500 | 1+ 1 | 99.0 ± 0.9 |

### [Adsorption (capture) of stem cells onto the prepared nonwoven fabrics]

Next, the prepared nonwoven fabrics were used as the stem cell adhesion sheets. A cell suspension solution containing human adipose-derived mesenchymal stem cells (ADSC; PromoCell, product code C-12977) was allowed to pass through the stem cell adhesion sheet being clamped by a filter sterilization holder, thereby human mesenchymal stem cells were attached to the stem cell adhesion sheet, and the adhesion rate was evaluated. Swinnex filter holder (Merck Millipore) was used for the filter sterilization holder. Further, the cell suspension solution was dripped dropwise one drop at a time. Experiments were conducted for three samples, respectively.

The results of attachment (capture) experiments are also indicated in Table 1. Note that the capture rate is a value found by measuring the number of cells before and after filtration by using a hemocytometer according to a formula: (the number of cells before filtration - the number of cells after filtration) / the number of cells before filtration x 100. As is clear from the results indicated in Table 1, it has been confirmed that the use of the stem cell adhesion sheet having the nonwoven fabric prepared in Example 1 enables attachment of stem cells at a significantly high efficiency.

### <Example 2>

A nonwoven fabric was prepared in the same procedure as in Example 1 except that the nonwoven fabric was prepared by using 100% polycaprolactone instead of the polymer material of Example 1. The sizes of the first layer and the second layer of the prepared nonwoven fabric were as follows. Further, the cell capture rate was 100%.
- First layer: fiber diameter was 0.5 µm, basis weight was 2 g/m², and thickness was 100 µm.
- Second layer: fiber diameter was 5 µm, basis weight was 10 g/m², and thickness was 500 µm.

### [Observation of growth factor production ability]

Next, a tissue regeneration promoting sheet was prepared by using the nonwoven fabric prepared in Example 2 to attach stem cells to the nonwoven fabric in the same procedure as in Example 1. The prepared tissue regeneration promoting sheet was transferred to a Petri dish containing a culture medium and subjected to culture for four weeks. Further, stem cells (ADSC) were directly cultured on a Petri dish for four weeks as the control. After the four-week culture, the expression level of vascular endothelial growth factor (VEGF) was measured by using ELISA. The results are illustrated in FIG. 6.

### <Example 3>

The growth factor production ability was observed by using the nonwoven fabric prepared in the same manner as in Example 2 except that the polymer material of Example 1 was used. The sizes of the first layer and the second layer of the prepared nonwoven fabric were as follows. Further, the cell capture rate was 99.7%. The results were illustrated in FIG. 7.
- First layer: fiber diameter was 0.5 µm, basis weight was 2 g/m², and thickness was 100 µm.
- Second layer: fiber diameter was 4 µm, basis weight was 10 g/m², and thickness was 500 µm.

As is clear from FIG. 6 and FIG. 7, it has been confirmed that, when stem cells were attached to the stem cell adhesion sheet disclosed in the present application and then cultured, the stem cells were more activated than when stem cells were directly cultured with a culture medium (ADSC in FIG. 6 and FIG. 7). Further, it has been confirmed in the series of experiments that, since the stem cell adhesion sheet disclosed in the present application has a laminated structure of the first layer and the second layer having a larger fiber diameter than the first layer, there is no particular problem in ease of handling of the stem cell adhesion sheet.

### <Example 4>

A nonwoven fabric was prepared in the same procedure as in Example 1 except that, instead of the polymer material of Example 1, a material obtained by copolymerizing polycaprolactone (PCL) and polylactic acid (PLA) at a ratio of 25:75, which has already been used clinically as absorbable sutures, was used as the polymer materials of the first layer 12 and the second layer 14, and adsorption (capture) of stem cells was performed. Table 2 lists the fiber diameters, the basis weights, and the thicknesses of the first layer and the second layer, the number of laminated layers, and the capture rate of stem cells.

**[Table 2]**

| First layer fiber diameter (*µ*m) | First layer basis weight (g/m²) | First layer thickness (*µ*m) | Second layer fiber diameter (*µ*m) | Second layer basis weight (g/m²) | Second layer thickness (*µ*m) | Number of laminated layers | Capture rate(%) |
|---|---|---|---|---|---|---|---|
| 0.5 | 2.2 | 110 | 4.5 | 5.5 | 275 | 1+ 1 | 98.7 ± 1.0 |

As is clear from the result indicated in Table 2, it has been confirmed that the use of the stem cell adhesion sheet having the nonwoven fabric prepared in Example 4 enables attachment of stem cells at a significantly high efficiency.

### <Comparative example 1>

Only polycaprolactone was used as the polymer material, and a nonwoven fabric having only the first layer was prepared. The size of the prepared nonwoven fabric was as follows.
- First layer: fiber diameter was 0.5 µm, basis weight was 2 g/m², and thickness was 100 µm.

Although the prepared nonwoven fabric was tried to be set to the holder, the nonwoven fabric was unable to be set to the holder because of poor handling.

### <Comparative example 2>

A nonwoven fabric was prepared in the same procedure as in Example 1 except that the polymer material described in Example 4 was used and that the fiber diameter, the basis weight. and the thickness listed in Table 3 below were employed and the number of laminated layers was one, and adsorption (capture) of stem cells was performed. The capture rate is also listed in Table 3 below.

**[Table 3]**

| Fiber diameter (*µ*m) | Basis weight (g/m²) | Thickness (*µ*m) | Number of laminated layers | Capture rate(%) |
|---|---|---|---|---|
| 4.5 | 2 | 110 | 1 | 43.2 ± 12.7 |
| 4.3 | 5.5 | 275 | 1 | 55.5 ± 14.7 |

As listed in Table 3, since the fiber diameter of the fibers forming the nonwoven fabric prepared in Comparative example 2 is a value very close to the fiber diameters of the second layer of Examples 1 to 4, there is no particular problem in the ease of handling even with substantially the same basis weight as that in Comparative example 1. Further, in Comparative example 2, while the capture rate of stem cells was lower than in Examples 1 to 4, a certain quantity of stem cells was able to be captured. Therefore, it has been confirmed that the second layer of the stem cell adhesion sheet disclosed in the present application achieves the adhesion (capture) function for stem cells and the reinforcement function of reinforcing the first layer. Note that it has been confirmed from the result indicated in Table 3 that, if a low capture rate of stem cells is not a problem, the fiber diameter being greater than a predetermined diameter allows the nonwoven fabric having even a single layer to function as the stem cell adhesion sheet.

From the results described above, it has been confirmed that, rather than preparing the stem cell adhesion sheet with only the first layer having a small fiber diameter, employing a laminated structure of the first layer and the second layer, which is formed of fibers having a larger fiber diameter than the first layer, improves the ease of handling and the capture efficiency of stem cells when stem cells are attached to the stem cell adhesion sheet.

### [List of Reference Symbols]

- 10: stem cell adhesion sheet
- 12: first layer
- 14: second layer
- 20: holder

## Claims

1. A stem cell adhesion sheet comprising a nonwoven fabric,
wherein the nonwoven fabric has a laminated structure including
a first layer formed of fibers mainly composed of a biocompatible polymer, and
a second layer formed of fibers mainly composed of a biocompatible polymer and formed of fibers having a larger fiber diameter than fibers forming the first layer, and
wherein the biocompatible polymer forming the first layer and the biocompatible polymer forming the second layer contain the same type of polymer material.

2. The stem cell adhesion sheet according to claim 1, wherein the polymer material is of at least one type selected from a group consisting of polylactic acid, polycaprolactone, chitin, chitosan, polyglycolic acid, and a copolymer thereof.

3. The stem cell adhesion sheet according to claim 2, wherein the polymer material is at least one type selected from a group consisting of polylactic acid, polycaprolactone, and a copolymer of polylactic acid and polycaprolactone.

4. The stem cell adhesion sheet according to claim 1, wherein polymer materials of the biocompatible polymer forming the first layer and the biocompatible polymer forming the second layer contain the same type of polymer material at a ratio of at least 25% or higher.

5. The stem cell adhesion sheet according to claim 2, wherein polymer materials of the biocompatible polymer forming the first layer and the biocompatible polymer forming the second layer contain the same type of polymer material at a ratio of at least 25% or higher.

6. The stem cell adhesion sheet according to claim 3, wherein polymer materials of the biocompatible polymer forming the first layer and the biocompatible polymer forming the second layer contain the same type of polymer material at a ratio of at least 25% or higher.

7. The stem cell adhesion sheet according to any one of claims 4 to 6, wherein the polymer materials of the biocompatible polymer forming the first layer and the biocompatible polymer forming the second layer are composed of the same type of material at 100%.

8. The stem cell adhesion sheet according to any one of claims 1 to 6,
wherein the fiber diameter of the fibers forming the first layer is in a range of 200 nm or greater and 5 µm or less, and
wherein the fiber diameter of the fibers forming the second layer is larger than the fiber diameter of the fibers forming the first layer and is in a range of 30 µm or less.

9. The stem cell adhesion sheet according to any one of claims 1 to 6,
wherein a basis weight of the first layer is in a range of 0.2 g/m² or greater and 4 g/m² or less, and
wherein a basis weight of the second layer is in a range of 2 g/m² or greater and 30 g/m² or less.

10. The stem cell adhesion sheet according to claim 3,
wherein inclusion of siloxane in the biocompatible polymer is excluded, and
wherein the fiber diameter of the fibers forming the second layer is 3.2 µm to 15 µm excluding that the fiber diameter is 4.0 µm or less and that the fiber diameter is 10 µm or greater.

11. The stem cell adhesion sheet according to claim 1,
wherein inclusion of siloxane in the biocompatible polymer is excluded,
wherein the fiber diameter of the fibers forming the first layer is 200 nm or greater and 5 µm or less excluding that the fiber diameter is 1.5 µm or greater, and
wherein the fiber diameter of the fibers forming the second layer is 3.2 µm to 15 µm excluding that the fiber diameter is 4.0 µm or less and that the fiber diameter is 10 µm or greater.

12. The stem cell adhesion sheet according to claim 1,
wherein inclusion of siloxane in the biocompatible polymer is excluded,
wherein a basis weight of the first layer is in a range of 0.2 g/m² or greater and 4 g/m² or less, and
wherein the fiber diameter of the fibers forming the second layer is 3.2 µm to 15 µm excluding that the fiber diameter is 4.0 µm or less and that the fiber diameter is 10 µm or greater.
